Europäisches Patentamt

European Patent Office

Office européen des brevets

(11) Numéro de publication : **0 435 705 B1**

(12) **FASCICULE DE BREVET EUROPEEN**

(19)

(45) Date de publication du fascicule du brevet :
**11.08.93 Bulletin 93/32**

(51) Int. Cl.⁵ : **C07C 19/08, C07C 17/00**

(21) Numéro de dépôt : **90403192.9**

(22) Date de dépôt : **09.11.90**

(54) **Hydrogénolyse sélective de dérivés perhalogénés de l'éthane.**

(30) Priorité : **15.12.89 FR 8916643**

(43) Date de publication de la demande :
**03.07.91 Bulletin 91/27**

(45) Mention de la délivrance du brevet :
**11.08.93 Bulletin 93/32**

(84) Etats contractants désignés :
**DE ES FR GB IT NL**

(56) Documents cités :
**EP-A- 0 379 396**
**GB-A- 2 210 880**
**US-A- 4 925 998**
**CHEMICAL ABSTRACTS, vol. 112, no. 7, 12**
**février 1990, page 685, résumé no. 54964s,**
**Columbus, Ohio, US; & JP-A-01 172 348**

(73) Titulaire : **ELF ATOCHEM S.A.**
**4 & 8, Cours Michelet La Défense 10**
**F-92800 Puteaux (FR)**

(72) Inventeur : **Cognion, Jean-Marie**
**85, Route de Charly**
**F-69230 Saint-Genis-Laval (FR)**
Inventeur : **Guillet, Dominique**
**2, Route de Vourlses, Bâtiment B**
**F-69230 Saint-Genis-Laval (FR)**

(74) Mandataire : **Leboulenger, Jean et al**
**ATOCHEM Département Propriété Industrielle**
**Cédex 42- La Défense 10**
**F-92091 Paris la Défense (FR)**

**Description**

La présente invention concerne la fabrication des chlorofluoroéthanes de formule:

$$CF_3-CHF_xCl_{2-x} \qquad (I)$$

où x est égal à 0 ou 1, par hydrogénation catalytique d'un perhalogéno-éthane de formule:

$$CF_3-CF_xCl_{3-x} \qquad (II)$$

Les deux matières premières, incluses dans la formule (II), sont le trichloro-1,1,1 trifluoro-2,2,2 éthane ($CF_3CCl_3$) et le dichloro-1,1 tétrafluoro-1,2,2,2 éthane ($CF_3CFCl_2$) dont la substitution d'un atone de chlore par un atome d'hydrogène conduit respectivement au dichloro-1,1 trifluoro-2,2,2 éthane ($CF_3CHCl_2$) et au chloro-1 tétrafluoro-1,2,2,2 éthane ($CF_3CHFCl$).

L'hydrogénation catalytique des composés (II) a déjà été décrite, mais les sélectivités en produit correspondant à l'élimination d'un seul atome de chlore sont faibles. Ainsi, l'hydrogénolyse du dichloro-1,1 tétrafluoro-1,2,2,2 éthane à 280°C sur un catalyseur à 5% de palladium sur charbon (brevet GB 1 578 933) fournit un produit contenant 70% de tétrafluoro-1,1,1,2 éthane. Des résultats semblables sont obtenus par C. GERVASUTTI et al (Journal of Fluorine Chemistry 1981, 1, 1-20) sur un catalyseur à 0,5% de palladium sur charbon: à 170°C, l'hydrogénolyse du dichloro-1,1 tétrafluoro-1,2,2,2 éthane conduit à 76% de tétrafluoro-1,1,1,2 éthane.

Des catalyseurs métalliques supportés sur charbon à 0,5% de rhénium, de platine ou de rhodium (publications JP 132536/89, 132537/89 et 132538/89 respectivement) ont également été testés dans l'hydrogénolyse du dichloro-1,1 tétrafluoro-1,2,2,2 éthane; ils conduisent à la formation importante de trifluoro-1,1,1 éthane qui peut atteindre 30% à 200°C. D'autre part, a également été décrite la déchloration totale du dichloro-1,1 tétrafluoro-1,2,2,2 éthane sur des catalyseurs bimétalliques à base de palladium (publication JP 172348/89) ou à base de platine (publications JP 132349/89 et 128942/89).

Pour résoudre le problème de l'élimination d'un seul atome de chlore, il faut recourir selon la demande de brevet japonais 106051/82 (publication JP 222038/83) à une réduction chimique par le couple zinc-éthanol; dans les conditions décrites, la sélectivité de l'hydrogénolyse du trichloro-1,1,1 trifluoro-2,2,2 éthane en dichloro-1,1 trifluoro-2,2,2 éthane atteint 90%. Cependant, ce procédé présente l'inconvénient d'employer du zinc métallique coûteux et de sous-produire du chlorure de zinc qu'il faut détruire.

Des résultats intéressants ont été obtenus dans l'hydrogénolyse catalytique avec des catalyseurs à 0,5% de platine sur alumine ou charbon (publication JP 149739/89): à 175°C avec un catalyseur sur charbon on obtient, à partir du trichloro-1,1,1 trifluoro-2,2,2 éthane, un produit contenant 64% de dichloro-1,1 trifluoro-2,2,2 éthane et à 200°C avec un catalyseur sur alumine le dichloro-1,1 tétrafluoro-1,2,2,2 éthane conduit à un produit contenant 42% de chloro-1 tétrafluoro-1,2,2,2 éthane.

Il a maintenant été trouvé que l'élimination catalytique d'un seul atome de chlore s'effectue très sélectivement si l'on utilise un catalyseur à base d'iridium.

La présente invention a donc pour objet un procédé de préparation des chlorofluoroéthanes de formule (I) par hydrogénation catalytique d'un perhalogéno-éthane de formule (II), caractérisé en ce que l'on emploie un catalyseur à base d'iridium déposé sur un support.

Dans le catalyseur utilisé selon l'invention, la teneur en iridium peut aller de 0,1 à 10% en poids, mais est de préférence comprise entre 0,2 et 8%.

Le support peut être de nature très diverse et choisi, par exemple, parmi les alumines, le fluorure d'aluminium et les charbons actifs. On préfère comme supports les charbons ayant une surface spécifique comprise entre 200 et 1500 m²/g (de préférence entre 600 et 1200 m²/g), une porosité élevée (0,3 à 0,7 cm³/g) et une granulométrie compatible avec une catalyse en lit fixe (1 à 10 mm). Ces produits sont commercialisés sous forme d'extrudés ou de billes par de nombreuses Sociétés.

Le catalyseur selon l'invention peut être préparé par imprégnation du support avec une solution aqueuse ou organique d'un dérivé de l'iridium, évaporation de l'eau ou du solvant et traitement thermique à une température allant de 150 à 500°C (de préférence 200 à 400°C) et sous courant d'hydrogène (de préférence sous une pression de 1 à 5 bars) pour libérer le métal. La nature du dérivé de l'iridium utilisé est sans importance et peut être, par exemple, un chlorure, l'acide chloroiridique ou son sel d'ammonium.

Le catalyseur selon l'invention peut également être choisi parmi les produits disponibles sur le marché, par exemple ceux de la Société ENGELHARD qui propose des catalyseurs contenant de 0,5 à 5% d'iridium sur des alumines ou des charbons.

L'hydrogénation catalytique selon l'invention peut être effectuée à une température allant de 50 à 300°C, de préférence comprise entre 150 et 250°C, avec un rapport molaire hydrogène/perhalogénoéthane (II) allant de 0,5 à 8 (de préférence 1 à 5), sous une pression de 1 à 20 bars (de préférence 1 à 5 bars) et un débit horaire de 1 à 20 moles de perhalogénoéthane (II) par litre de catalyseur.

Les exemples suivants illustrent l'invention sans la limiter. Dans les exemples 2 à 8, les résultats sont exprimés par le taux de transformation globale (TT$_G$) et la sélectivité (S) en un produit de la réaction:

$$TT_G = 100 \times \frac{\text{Nombre de moles de composé (II) transformé}}{\text{Nombre de moles de composé (II) introduit}}$$

$$S = 100 \times \frac{\text{Nombre de moles de produit formé}}{\text{Nombre de moles de composé (II) transformé}}$$

les analyses à l'entrée et en sortie du réacteur étant effectuées par chromatographie phase vapeur en ligne.

## EXEMPLE 1 : Préparation des catalyseurs

### Catalyseur A

Dans un évaporateur rotatif, on charge 60 ml (28 g) d'un charbon actif ayant une porosité de 0,6 cm³/g et une surface spécifique de 950 m²/g sous forme d'extrudés de 1,8 mm de diamètre. Après dégazage pendant 3 heures à 100°C sous pression réduite (1 kPa), on introduit 70 ml d'une solution aqueuse de trichlorure d'iridium hydraté (53,3% d'Ir) contenant 2,6 g de IrCl$_3$, puis on évapore l'eau sous pression réduite (1 kPa) et sèche à 100°C. On traite ensuite à 400°C pendant 2 heures sous un courant d'hydrogène (10 Nl/h) et obtient ainsi un catalyseur A contenant 5% d'iridium.

### Catalyseur B

En opérant de la même façon, mais avec une solution aqueuse contenant 0,53 g de IrCl$_3$, on obtient un catalyseur B à 1% d'iridium.

### Catalyseur C

On opère de la même façon que pour préparer le catalyseur B, sauf que le traitement à l'hydrogène est effectué à 200°C au lieu de 400°C. Le catalyseur C ainsi obtenu contient aussi 1% d'iridium.

### Catalyseur D

On opère de la même façon que pour préparer le catalyseur A, mais on remplace le charbon actif par le même volume d'une alumine ayant une porosité de 0,48 cm³/g et une surface spécifique de 129 m²/g sous forme de sphères de 2 mm de diamètre et on utilise une solution aqueuse de chlorure d'iridium contenant 0,35 g d'IrCl$_3$. On obtient ainsi un catalyseur D contenant 0,5% d'iridium.

## EXEMPLE 2

Dans un tube en Inconel de 45 cm de long et de 2,72 cm de diamètre intérieur, chauffé électriquement, on introduit 50 ml du catalyseur A décrit à l'exemple 1, puis on y fait passer un mélange d'hydrogène et de dichloro-1,1 tétrafluoro-1,2,2,2 éthane aux rapports molaires, débits et températures indiqués dans le tableau suivant dont la dernière partie rassemble les résultats obtenus.

## TABLEAU 1

| ESSAI N° | 1 | 2 | 3 | 4 |
|---|---|---|---|---|
| **Conditions opératoires :** | | | | |
| Température (°C) | 100 | 130 | 150 | 200 |
| Rapport molaire $H_2/C_2F_4Cl_2$ | 5 | 5 | 5 | 5 |
| Débit $C_2F_4Cl_2$ (mole/heure) | 0,05 | 0,05 | 0,05 | 0,05 |
| **Résultats** | | | | |
| % $TT_G$ du $C_2F_4Cl_2$ | 4 | 42 | 97 | 100 |
| % S en $CF_3CHFCl$ | 86 | 34 | 74 | 50 |
| % S en $CF_3CH_2F$ | 9 | 9 | 13 | 20 |
| % S en $CF_3CH_3$ | 4 | 6 | 13 | 30 |

Dans la plupart des cas, la sélectivité de l'hydrogénolyse d'une seule liaison C-Cl est supérieure à 70%.

A titre comparatif, on a effectué deux essais en remplaçant le catalyseur A selon l'invention par un catalyseur à 5% de palladium préparé de la même façon et sur le même support qu'à l'exemple 1 avec $PdCl_2$ au lieu de $IrCl_3$. Les résultats, rassemblés dans le tableau 2 suivant, montrent qu'avec ce catalyseur au palladium, la sélectivité de la réaction est nettement en faveur de l'arrachement de deux atomes de chlore.

TABLEAU 2

| ESSAI COMPARATIF N° | 5 | 6 |
|---|---|---|
| Conditions opératoires : | | |
| Température (°C) | 150 | 200 |
| Rapport molaire $H_2/C_2F_4Cl_2$ | 4 | 4 |
| Débit $C_2F_4Cl_2$ (mole/heure) | 0,07 | 0,07 |
| Résultats : | | |
| % $TT_G$ du $C_2F_4Cl_2$ | 100 | 100 |
| % S en $CF_3CHFCl$ | 4 | 3 |
| % S en $CF_3CH_3$ | 1 | 1,2 |
| % S en $CF_3CH_2F$ | 94,5 | 95 |

**EXEMPLE 3**

Dans le même appareillage qu'à l'exemple 2 et avec une charge de 25 ml de catalyseur A, on effectue à nouveau des essais d'hydrogénolyse du dichloro-1,1 tétrafluoro-1,2,2,2 éthane en diminuant le rapport molaire $H_2/CF_3CFCl_2$.

Les conditions opératoires des essais et les résultats obtenus sont rassemblés dans le tableau 3 suivant.

**TABLEAU 3**

| ESSAI N° | 7 | 8 | 9 |
|---|---|---|---|
| **Conditions opératoires :** | | | |
| Température (°C) | 103 | 153 | 200 |
| Rapport molaire $H_2/C_2F_4Cl_2$ | 1,5 | 1,5 | 1,5 |
| Débit $C_2F_4Cl_2$ (mole/heure) | 0,07 | 0,07 | 0,07 |
| **Résultats :** | | | |
| % $TT_G$ du $C_2F_4Cl_2$ | 26 | 62 | 97 |
| % S en $CF_3CHFCl$ | 88 | 79 | 76 |
| % S en $CF_3CH_3$ | 6 | 12 | 17 |
| % S en $CF_3CH_2F$ | 6 | 7 | 7 |

**EXEMPLE 4-7**

Dans le même appareillage qu'à l'exemple 2 et avec une charge de 25 ml de catalyseur, on a procédé à différents essais d'hydrogénolyse du dichloro-1,1 tétrafluoro-1,2,2,2 éthane en utilisant les catalyseurs B, C et D.

Les conditions opératoires et les résultats de ces essais sont rassemblés dans le tableau 4 suivant.

## TABLEAU 4

| EXEMPLE | 4 | 5 | 6 | 7 |
|---|---|---|---|---|
| Conditions opératoires : | | | | |
| Catalyseur | B | C | C | D |
| Température (°C) | 150 | 150 | 190 | 230 |
| Rapport molaire $H_2/C_2F_4Cl_2$ | 4,5 | 5 | 5 | 1,5 |
| Débit $C_2F_4Cl_2$ (mole/heure) | 0,03 | 0,05 | 0,05 | 0,07 |
| Résultats : | | | | |
| % $TT_G$ du $C_2F_4Cl_2$ | 55 | 59 | 83 | 97 |
| % S en $CF_3CHFCl$ | 81 | 90 | 92 | 76 |
| % S en $CF_3CH_3$ | 11 | 6 | 4 | 14 |
| % S en $CF_3CH_2F$ | 8 | 4 | 4 | 6 |

## EXEMPLE 8

Dans le même appareillage qu'à l'exemple 2, on introduit 50 ml d'une nouvelle charge de catalyseur A sur laquelle on effectue l'hydrogénation du trichloro-1,1,1 trifluoro-2,2,2 éthane ($CF_3$-$CCl_3$).

Les conditions opératoires de l'essai et les résultats obtenus sont rassemblés dans le tableau 5 suivant. A côté du produit attendu, le dichloro-1,1 trifluoro-2,2,2 éthane ($CF_3CHCl_2$), on trouve principalement comme sous-produit le trifluoro-1,1,1 éthane ($CF_3CH_3$) et le chloro-1 trifluoro-2,2,2 éthane ($CF_3CH_2Cl$).

EP 0 435 705 B1

## TABLEAU 5

| Conditions opératoires : | |
|---|---|
| Température (°C) | 160 |
| Rapport molaire $H_2/C_2F_3Cl_3$ | 1,5 |
| Débit $C_2F_3Cl_3$ (mole/heure) | 0,06 |
| Résultats : | |
| % $TT_G$ du $C_2F_3Cl_3$ | 68 |
| % S en $CF_3CHCl_2$ | 81 |
| % S en $CF_3CH_3$ | 8 |
| % S en $CF_3CH_2Cl$ | 3 |

**Revendications**

1. Procédé de préparation des chlorofluoroéthanes de formule:
$$CF_3\text{-}CHF_xCl_{2\text{-}x} \qquad (I)$$
où x est égal à 0 ou 1, par hydrogénation catalytique d'un perhalogénoéthane de formule:
$$CF_3\text{-}CF_xCl_{3\text{-}x} \qquad (II)$$
caractérisé en ce qu'on utilise un catalyseur à base d'iridium déposé sur un support.

2. Procédé selon la revendication 1, caractérisé en ce que le catalyseur a une teneur en iridium allant de 0,1 à 10% en poids, de préférence entre 0,2 et 8%.

3. Procédé selon la revendication 1 ou 2, caractérisé en ce que le support est une alumine, le fluorure d'aluminium ou un charbon actif.

4. Procédé selon la revendication 1 ou 2, caractérisé en ce que le support est un charbon actif ayant une surface spécifique comprise entre 200 et 1500 m2/g, de préférence entre 600 et 1200 m2/g, une porosité de 0,3 à 0,7 cm3/g et une granulométrie de 1 à 10 mm.

5. Procédé selon l'une des revendications 1 à 4, dans lequel l'hydrogénation est effectuée à une température comprise entre 50 et 300°C, de préférence entre 150 et 250°C et sous une pression de 1 à 20 bars, de préférence 1 à 5 bars.

6. Procédé selon l'une des revendications 1 à 5, dans lequel le rapport molaire hydrogène/perhalogénoéthane (II) est compris entre 0,5 et 8, de préférence entre 1 et 5.

7. Pocédé selon l'une des revendications 1 à 6, dans lequel le débit horaire de perhalogénoéthane (II) est de 1 à 20 moles par litre de catalyseur.

8

**Claims**

1. Process for preparing chlorofluoroethanes of the formula:
$$CF_3\text{-}CHF_xCl_{2-x} \qquad (I)$$
where x is equal to 0 or 1, by the catalytic hydrogenation of a perhaloethane of the formula:
$$CF_3\text{-}CF_xCl_{3-x} \qquad (II)$$
characterised in that an iridium-based catalyst deposited on a support is used.

2. Process according to Claim 1, characterised in that the catalyst has an iridium content ranging from 0.1 to 10% by weight, and preferably between 0.2 and 8%.

3. Process according to Claim 1 or 2, characterised in that the support is an alumina, aluminium fluoride or an active charcoal.

4. Process according to Claim 1 or 2, characterised in that the support is an active charcoal having a specific surface area of between 200 and 1500 $m^2$/g, preferably of between 600 and 1200 $m^2$/g, a porosity of 0.3 to 0.7 $cm^3$/g and a particle size of 1 to 10 mm.

5. Process according to one of Claims 1 to 4, in which the hydrogenation is performed at a temperature of between 50 and 300°C, preferably of between 150 and 250°C, and under a pressure of 1 to 20 bars, preferably 1 to 5 bars.

6. Process according to one of Claims 1 to 5, in which the mole ratio hydrogen/perhaloethane (II) is between 0.5 and 8, preferably between 1 and 5.

7. Process according to one of Claims 1 to 6, in which the hourly flow rate of perhaloethane (II) is from 1 to 20 moles per litre of catalyst.

**Patentansprüche**

1. Verfahren zur Herstellung von Chlorfluorethanen der Formel:
$$CF_3\text{-}CHF_xCl_{2-x} \qquad (I)$$
in der x gleich 0 oder 1 ist, durch katalytische Hydrierung eines Perhalogenethans der Formel:
$$CF_3\text{-}CF_xCl_{3-x} \qquad (II)$$
dadurch gekennzeichnet, daß ein Katalysator auf Iridiumbasis verwendet wird, der auf einem Träger aufgebracht ist.

2. Verfahren nach Anspruch 1, dadurch gekennzeichnet, daß der Katalysator einen Iridiumgehalt zwischen 0,1 und 10 Gewichtsprozenten, bevorzugt zwischen 0,2 und 8 %, besitzt.

3. Verfahren nach Anspruch 1 oder 2, dadurch gekennzeichnet, daß der Träger aus Aluminiumoxid, Aluminiumfluorid oder Aktivkohle besteht.

4. Verfahren nach Anspruch 1 oder 2, dadurch gekennzeichnet, daß der Aktivkohle-Träger eine spezifische Oberfläche zwischen 200 und 1500 $m^2$/g, bevorzugt zwischen 600 und 1200 $m^2$/g, eine Porosität zwischen 0,3 und 0,7 $cm^3$/g und eine Korngröße zwischen 1 und 10 mm aufweist.

5. Verfahren nach einem der Ansprüche 1 bis 4, in dem die Hydrierung bei einer Temperatur zwischen 50 und 300°C, bevorzugt zwischen 150 und 250°C, und bei einem Druck zwischen 1 und 20 bar, bevorzugt zwischen 1 bis 5 bar, durchgeführt wird.

6. Verfahren nach einem der Ansprüche 1 bis 5, in dem das Molverhältnis von Wasserstoff zu Perhalogenethan (II) zwischen 0,5 und 8, bevorzugt zwischen 1 und 5, liegt.

7. Verfahren nach einem der Ansprüche 1 bis 6, in dem der stündliche Durchsatz an Perhalogenethan (II) zwischen 1 und 20 Mol pro Liter Katalysator liegt.